## Europäisches Patentamt

## European Patent Office

## Office européen des brevets

(19)

(11) Numéro de publication: **0 033 687**
**B1**

(12) **FASCICULE DE BREVET EUROPEEN**

(45) Date de publication du fascicule du brevet:
**09.11.83**

(21) Numéro de dépôt: **81400121.0**

(22) Date de dépôt: **28.01.81**

(51) Int. Cl.³: **A 61 K 7/135**

(54) Procédé de pré-conditionnement des composants d'un mélange pour la décoloration des cheveux, et composants ainsi obtenus.

(30) Priorité: **30.01.80 FR 8001999**

(43) Date de publication de la demande:
**12.08.81 Bulletin 81/32**

(45) Mention de la délivrance du brevet:
**09.11.83 Bulletin 83/45**

(84) Etats contractants désignés:
**AT BE CH DE FR GB IT LI LU NL SE**

(56) Documents cités:
**DE - A - 2 013 762**
**DE - A - 2 205 461**
**DE - B - 1 266 283**

**Le dossier contient des informations techniques présentées postérieurement au dépôt de la demande et ne figurant pas dans le présent fascicule.**

(73) Titulaire: **Perma, 29bis, rue d'Astorg, F-75008 Paris (FR)**

(72) Inventeur: **Froger, Hubert, 22-26, rue Saint-Denis, F-77400 Lagny-Sur-Marne (FR)**

(74) Mandataire: **Le Brusque, Maurice, CREUSOT-LOIRE 15 rue Pasquier, F-75383 Paris Cedex 08 (FR)**

BUNDESDRUCKEREI BERLIN

Produit décolorant pour cheveux

La présente invention concerne un produit décolorant pour cheveux constitué de deux composants séparés, l'un fluide, l'autre solide, destinés à la préparation, au moment de l'usage, d'un mélange final instable contenant de l'eau oxygénée.

Dans la quasi totalité des cas, la décoloration des cheveux dans les salons de coiffure est réalisée par oxydation alcaline, c'est-à-dire en faisant agir sur les cheveux de l'oxygène naissant en milieu alcalin.

L'eau oxygénée peut se présenter sous forme d'une pâte consistante obtenue par mélange d'une solution aqueuse avec un produit de base pulvérulent.

Dans le brevet DE.A.1266283, par exemple, on utilise comme base une substance anorganique comme de la silice. Cependant, pour que la pâte ait une consistance stable, on lui ajoute d'autres produits à réaction basique comme des sels alcalins.

Une telle pâte, assez stable pour être préparée à l'avance, n'est pas normalement destinée à la décoloration des cheveux car, pour être suffisemment efficase, le produit destiné à libérer l'oxygène naissant doit être instable. C'est pourquoi, dans ce cas, on a l'habitude d'employer un produit constitué de deux composants séparés, d'un côté l'eau oxygénée et de l'autre des additifs, le mélange n'étant préparé qu'immédiatement avant l'emploi.

Pour faciliter le dosage et le mélange des composants, il est préférable que l'eau oxygénée soit sous forme liquide, ou en tout cas fluide, les additifs se présentant sous forme de poudre.

Depuis longtemps, les poudres utilisées en association avec l'eau oxygénée sont généralement:

— des persels, comme des persulfates alcalins ou parfois des percarbonates ou peroxydes,
— des agents alcalins, comme des métasilicates ou parfois des phosphates ou carbonates,
— des charges minérales, comme du carbonate de magnésie ou de la magnésie, dont le but est de régulariser le dégagement d'oxygène naissant après mélange avec l'eau oxygénée.

Pour procéder à une décoloration, le coiffeur dispose donc actuellement de deux composants séparés, l'un fluide, l'autre solide, contenant respectivement:

— d'une part de l'eau oxygénée, généralement à vingt volumes et stabilisée en milieu acide,
— d'autre part l'ensemble des produits destinés à être mélangés à l'eau oxygénée et présentés par le fabricant spécialisé sous forme d'un mélange de diverses poudres en proportions relatives convenables.

Juste avant la décoloration, le coiffeur procède donc au mélange final en prélevant une quantité dosée dans le récipient d'eau oxygénée, et une quantité dosée dans le récipient contenant les poudres prémélangées; par mélange des deux doses il obtient une pâte qui est appliquée sur les cheveux.

Pour éviter l'inconvénient d'un séchage rapide sur la tête, et le désagrément correspondant d'une impression de tiraillement, on a plus récemment ajouté dans les poudres de la silice colloïdale qui présente l'avantage d'améliorer la fluidité de la poudre, d'améliorer l'adhérence de la pâte et de mieux régulariser le dégagement d'oxygène naissant. On obtient ainsi un gel qui donne une meilleure onctuosité à la pâte et facilite son application et sa tenue sur le cheveu où elle doit pouvoir continuer à agir pendant une durée de l'ordre d'une demi-heure à une heure.

Or on a constaté que l'adjonction de silice colloïdale dans les poudres, qui améliore grandement l'application et la localisation de la pâte, présente par contre un grave inconvénient. En effet, la silice colloïdale très pure utilisée présente une densité très faible, de l'ordre de 50 grammes par litre, et une surface spécifique élevée, de l'ordre de 100 m2 par gramme. Ces caractéristiques rendent très difficile son incorporation homogène aux autres poudres dès que l'on recherche une concentration supérieure à 0,1 à 0,5% alors que pour obtenir ensuite un gel avec l'eau oxygénée il faut un taux minimum de 5 à 10%.

Dans ces conditions, même si l'on a pu obtenir un mélange homogène initial lors de l'élaboration dans les ateliers du fabricant, les secousses lors des transports et manutentions des emballages entre l'usine d'élaboration et le salon de coiffure où ils seront utilisés, et même les manutentions chez l'utilisateur, entrainent une sédimentation systématique; la poudre n'est plus homogène dans son emballage au moment du prélèvement pour la préparation finale, si bien que la pâte obtenue ne l'est pas non plus, et l'on aboutit à des défauts de régularité des décolorations obtenues. Il n'a pas été possible jusqu'à présent de trouver un moyen pour empêcher cette sédimentation spontanée.

En outre l'empâtage final avec l'eau oxygénée se révèle difficile à obtenir, et exige un temps de malaxage important pour l'obtention de la pâte à appliquer. On observera encore que lors de ce mélange final, la poudre de silice extrêmement légère a tendance à voler hors du récipient de mélange, ce qui entraine des risques d'inhalation par le préparateur.

La présente invention permet, par une nouvelle présentation des composants mis à la disposition de l'utilisateur pour la préparation du produit final à appliquer, de rendre très facile, très rapide et sans danger l'obtention d'une pâte parfaitement homogène.

Selon l'invention, la silice colloïdale est incluse dans le composant fluide contenant l'eau oxygénée et le composant solide est constitué uniquement d'un mélange homogène des autres produits sous forme de poudres de granulométries et de densités voisines.

On voit que l'invention ne modifie pas la composition de la pâte formée par le mélange final réalisé juste avant l'application sur les cheveux, mais modifie la composition des deux composants servant à la préparation de ce mélange final. Elle résulte du fait d'avoir pu établir qu'il était possible, compte tenu de l'acidité de la phase aqueuse de l'eau oxygénée, d'y introduire progressivement et sous agitation toute la silice colloïdale nécessaire au mélange final, en obtenant cependant un liquide fluide, stable tant vis à vis de la décantation que du taux en oxygène.

En effet, on a observé que la silice ne se déposait pas lorsque sa teneur en poids restait comprise entre 2 et 15% et, de préférence, de l'ordre de 8%.

D'ailleurs, du fait que la silice est incluse dans un liquide, le risque de décantation présente moins d'inconvénient car il suffit que le coiffeur agite le flacon d'eau oxygénée avant l'emploi pour remettre la silice en suspension. Au contraire, lorsque la silice était incluse à la poudre, il n'était pas possible, en cas de sédimentation, de rétablir par une simple agitation, l'homogénéité du mélange. Dans le cas de l'invention, le reste de la poudre, sans silice, présente pour tous les produits, des granulométries et des densités voisines, si bien que son homogénéité obtenue lors de son élaboration se conserve facilement jusque sur la table de l'utilisateur.

On a en outre observé que le mélange final entre d'une part l'eau oxygénée chargée de silice, et d'autre part la poudre contenant les autres produits déjà mélangés, se faisait avec une remarquable facilité et très rapidement.

Pour éviter la décantation de la silice, il est également possible d'améliorer le produit en introduisant aussi, dans la phase aqueuse constituée par l'eau oxygénée chargée de silice, des additifs organiques tels que des gommes ou tensio-actifs.

Ces additifs, lorsqu'ils étaient prévus dans le mélange final, étaient jusqu' à présent prémélangés avec les autres poudres de persels, d'agents alcalins ou de charges minérales. En incorporant ces additifs organiques dans l'eau oxygénée par petites fractions et sous agitation, on obtient finalement une crème gélifiée très stable, qui permet un empâtage final encore plus facile avec le mélange des autres poudres fournies séparément, et conduit à une pâte très onctueuse et d'application particulièrement facile.

La réalisation du composant eau oxygénée-silice-additifs organiques présente en outre l'avantage d'éviter d'avoir à regrouper dans un même malaxeur de mélange des produits comme les persels et les constituants organiques, ce qui dans certaines conditions entrainait des risques de combustion spontanée et par conséquent de graves perturbations dans le processus industriel d'élaboration de ces mélanges. Dans la nouvelle formule du composant pulvérulent, seuls restent en présence des sels minéraux dont le malaxage n'impose aucune précaution particulière.

Bien entendu l'invention n'est pas strictement limitée à ce qui a été décrit à titre d'exemple, mais elle en couvre également les variantes de détail ou résultant de l'utilisation de moyens équivalents.

**Revendications**

1. Produit décolorant pour cheveux constitué de deux composants séparés, l'un fluide l'autre solide, destinés à la préparation, au moment de l'usage, d'un mélange final instable contenant de l'eau oxygénée, de la silice colloïdale, des persels, des agents alcalins, des additifs organiques et/ou des colorants, le composant fluide contenant l'eau oxygénée et le composant solide se présentant sous forme d'un mélange pulvérulent, caractérisé par le fait que la silice colloîdale est incluse en proportion de 2 à 15% en poids dans le composant fluide contenant l'eau oxygénée et que le composant solide est constitué uniquement d'un mélange homogène des autres produits sous forme de poudres de granulométries et de densités voisines.

2. Produit décolorant pour cheveux selon la revendication 1, caractérisé par le fait que le composant fluide contient des additifs organiques tels que des gommes organiques ou un tensio-actif.

**Patentansprüche**

1. Haarentfärbungsmittel, das aus zwei getrennten Bestandteilen besteht, von welchen einer flüssig und der andere fest ist und die dazu bestimmt sind, im Augenblick der Verwendung eine endgültige, instabile Mischung herzustellen, welche Wasserstoffperoxid, kolloidales Siliziumdioxid, Persalze, alkalische Mittel, organische Zusatzstoffe und/oder Farbstoffe enthält, wobei der flüssige Bestandteil Wasserstoffperoxid enthält und der feste Bestandteil eine pulverförmige Mischung ist, dadurch gekennzeichnet, daß das kolloidale Siliziumdioxid in einem Mengenverhältnis von 2—15 Gew.-% in den flüssigen, Wasserstoffperoxid enthaltenden Bestandteil eingebracht wird und daß der feste Bestandteil ausschließlich aus einer homogenen Mischung der anderen Substanzen in Form von Pulvern mit ähnlicher Korngrößenverteilung und Dichte besteht.

2. Haarentfärbungsmittel nach Anspruch 1, dadurch gekennzeichnet, daß der flüssige Bestandteil organische Zusatzstoffe wie organische Gummi oder ein oberflächenaktives Mittel enthält.

## Claims

1. Hair-bleaching product consisting of two separate components, the one fluid and the other solid, intended for the preparation, at the time of use, of an unstable final mixture containing hydrogen peroxide, colloidal silica, per-salts, alkaline agents, organic additives and/or dyestuffs, the fluid component containing the hydrogen peroxide and the solid component being presented in the form of a pulverulent mixture, characterised in that the colloidal silica is included, in a proportion of 2 to 15% by weight, in the fluid component containing the hydrogen peroxide, and in that the solid component consists solely of a homogeneous mixture of the other products in the form of powders of similar particle sizes and densities.

2. Hair-bleaching product according to Claim 1, characterised in that the fluid component contains organic additives such as organic gums or a surface-active agent.